# EUROPÄISCHE PATENTSCHRIFT

(11) **EP 4 251 594 B1**
(45) Veröffentlichungstag und Bekanntmachung des Hinweises auf die Patenterteilung: **31.07.2024**
(21) Anmeldenummer: 22701944.5
(22) Anmeldetag: 21.01.2022
(51) Int. Cl.: B01D 3/00, B01D 3/14, B01D 3/40, C07C 7/08, C07C 11/08

(54) **VERFAHREN ZUR VERHINDERUNG EINER DREIPHASIGKEIT BEI DER ABTRENNUNG VON BUTENEN AUS C4-KOHLENWASSERSTOFFSTRÖMEN**
METHOD FOR PREVENTING A THREE-PHASE SITUATION IN THE SEPARATION OF BUTENES FROM C4 HYDROCARBON FLOWS
PROCÉDÉ PERMETTANT D'ÉVITER TROIS ÉTAPES DE LA SÉPARATION DES BUTÈNES DES FLUX D'HYDROCARBURE C4

(30) Priorität: 27.01.2021 EP 21153659
(43) Veröffentlichungstag der Anmeldung: 04.10.2023
(73) Patentinhaber: Evonik Oxeno GmbH & Co. KG, 45772 Marl (DE)
(72) Erfinder: LUTZE, Philip, 46535 Dinslaken (DE); PEITZ, Stephan, 45739 Oer-Erkenschwick (DE); RIX, Armin Matthias, 45770 Marl (DE); SIX, Tanita Valèrie, 44309 Dortmund (DE); SCHRÖDER, Moritz, 48153 Münster (DE); PAUL, Niklas, 45770 Marl (DE)
(74) Vertreter: Evonik Patent Association
(86) Internationale Anmeldenummer: PCT/EP2022/051334
(87) Internationale Veröffentlichungsnummer: WO 2022/161864

(56) Entgegenhaltungen:
- US-A- 2 750 435
- US-A1- 2013 178 684
- US-A1- 2014 124 358

## Beschreibung

Die vorliegende Erfindung betrifft ein Verfahren zur Abtrennung von Butenen aus C4-Kohlenwasserstoffströmen, die neben den Butenen auch Butane enthält, durch Extraktivdestillation mit einem geeigneten Lösemittel. Das erfindungsgemäße Verfahren zeichnet sich durch eine Wärmeintegration aus, mit der die Wärme des Lösemittels zur Erwärmung verschiedener Ströme genutzt wird.

Die Trennung von Butan-Buten-Gemischen mittels Extraktivdestillation ist an sich bekannt. Dabei wird ein aprotisches Lösemittel (z. B. N-Methyl-2-pyrrolidon (NMP) oder Acetonitril (ACN)) eingesetzt, um die relative Flüchtigkeit der Alkane zu erhöhen. In einer Extraktivdestillationskolonne, dem Absorber, werden bevorzugt die Butene im Lösemittel gelöst, die Butane werden als Kopfprodukt abgetrennt. Das beladene Lösemittel wird anschließend in einer Stripkolonne, dem Desorber, bei erhöhter Temperatur von den Butenen befreit, die als Kopfprodukt in angereicherter Form gewonnen werden. Das von Butenen befreite Lösemittel wird dann zur Extraktivdestillation zurückgefahren.

Aufgrund des hohen Lösemittel-zu-Feed-Verhältnisses kommt der Wärmeintegration eine hohe Bedeutung für die Wirtschaftlichkeit des Verfahrens zu. Im Sumpf des Desorbers fällt heißes Lösemittel an, dessen Energieinhalt auf verschiedene Weise genutzt werden kann. In der

US 2014/0124358 A1 wird ein Verfahren zur selektiven Extraktion von Olefinen vorgestellt, welches die Aufgabe der Wärmeintegration lösen soll. Darin wird vorgeschlagen, den Energiegehalt des heißen Lösemittel zur Erwärmung eines Seitenstroms aus dem Desorber, zur Erwärmung des Sumpfprodukts des Absorbers, das zum Desorber geleitet wird, zur Erwärmung von einem oder mehreren Seitenströmen des Absorbers, und zur Vorwärmung des Feedstroms zu nutzen.

Bei der Trennung von Buten-Butan-Gemischen ist neben der Energierückgewinnung auch die Verhinderung einer Dreiphasigkeit im Absorber ein wichtiges Merkmal. Im Absorber geht ein an Butanen angereicherter Strom über Kopf. Um zu vermeiden, dass auch Teile des Lösemittels über den Kopf gehen, muss das Lösemittel zurückgewaschen werden. Dazu wird eine gewisse Menge an Rücklauf, der hohe Anteil an n-Butan enthalten kann, in eine Rückwaschzone im oberen Teil des Absorbers gefahren. Mit hohen n-Butan-Anteilen wird das Auftreten einer zweiten flüssigen Phase im Absorber jedoch wahrscheinlicher, wodurch nicht nur die Trennaufgabe des Absorbers erschwert wird, sondern auch zu Schaumproblemen (Ross-type foaming) führen kann. Zusätzlich muss betrachtet werden, dass je nach Einbindung der Buten-Butan-Trennung in einen chemischen Verbund, ein höherer n-Butananteil im Feedstrom zur Butan-Buten-Trennung vorhanden sein kann. Hohe n-Butananteile im Feedstrom verschieben aber die Gleichgewichtslage des n-Butans im Lösemittel, was dann wiederum die Wahrscheinlichkeit für das Auftreten einer zweiten flüssigen Phase erhöht.

Die Aufgabe der vorliegenden Erfindung bestand deshalb darin ein Verfahren bereitzustellen, bei dem das Auftreten einer zweiten flüssigen Phase und damit die Wahrscheinlichkeit einer Schaumbildung verringert werden kann. Weiterhin war die Aufgabe der vorliegenden Erfindung eine Steigerung der Trenneffizienz der Butan-Buten-Trennung und eine optimale Ausnutzung der Energie.

Diese Aufgabe kann durch die in Anspruch 1 vorgeschlagene Ausführung des Verfahrens gelöst werden. Bevorzugte Ausführungsformen sind in den Unteransprüchen angegeben. Das erfindungsgemäße Verfahren ist ein Verfahren zur Abtrennung von Butenen aus einem C4-Kohlenwasserstoffstrom, der zumindest Butene und Butane enthält, durch Extraktivdestillation mit einem Lösemittel, wobei das Verfahren die folgenden Schritte umfasst:
a. Zumindest teilweises Verdampfen des flüssigen C4-Kohlenwasserstoffstroms in einem Feedverdampfer, Zuführen des gasförmigen C4-Kohlenwasserstoffstroms und Zuführen des flüssigen Lösemittels zu einem Flüssigkeitsverteiler oberhalb des C4-Kohlenwasserstoffstroms zu einem Absorber, der mehr als zwei Füllkörperbetten umfasst und in dem der C4-Kohlenwasserstoffstrom und das Lösemittel miteinander in Kontakt gebracht werden, wodurch überwiegend Butene aus dem C4-Kohlenwasserstoffstrom in das Lösemittel übergehen, wobei das so beladene Lösemittel in einem Flüssigkeitssammler des Absorbers gesammelt, durch einen Absorberverdampfer gefahren und dann unterhalb des Flüssigkeitssammlers in den Sumpf des Absorbers geleitet wird, wodurch überwiegend Butane aus dem beladenen Lösemittel ausgegast werden, wobei das beladene Lösemittel anschließend als Sumpfstrom zu einem Desorber geführt wird und wobei am Kopf des Absorbers ein im Vergleich zum eingesetzten C4-Kohlenwasserstoffstrom an Butanen angereicherter Strom anfällt, der zumindest teilwiese kondensiert und ein Teil des Kondensats als Rücklauf zum Kopf des Absorbers geleitet wird;
b. Zuführen des beladenen Lösemittels, vorzugsweise NMP, zu dem Desorber, in dem im Vergleich mit dem Absorber eine erhöhte Temperatur und bevorzugt ein geringerer Druck vorliegt und in dem die Butene vom Lösemittel, vorzugsweise NMP, getrennt werden, wodurch am Kopf des Desorbers ein an Butenen angereicherter Strom und im Sumpf des Desorbers das zumindest teilweise von Butenen befreite Lösemittel, vorzugsweise NMP, anfällt, wobei das zumindest teilweise von Butenen befreite Lösemittel, vorzugsweise NMP, in einem Flüssigkeitssammler des Desorbers gesammelt, durch einen Desorberverdampfer gefahren und dann unterhalb des Flüssigkeitssammlers in den Sumpf des Desorbers geleitet wird, wodurch noch im Lösemittel vorhandene Butene ausgegast werden, und wobei das Lösemittel, vorzugsweise NMP, anschließend als Sumpfstrom zum Absorber zurückgeführt wird;
wobei die Wärme des als Sumpfstrom des Desorbers abgenommen Lösemittels zur Wärmeintegration verwendet wird, in dem die Wärme des Lösemittels in jeweils mindestens einem Wärmetauscher für die Vorwärmung des zum Desorber geführten beladenen Lösemittels, zur Verdampfung im Absorberverdampfer und zur Verdampfung des flüssigen C4-Kohlenwasserstoffstroms eingesetzt wird, und wobei
zumindest ein Teil einer flüssigen Phase am Flüssigkeitsverteiler oberhalb des ersten oder zweiten Füllkörperbettes abgenommen und in einem Dekanter in eine schwere flüssige Phase und eine leichte flüssige Phase aufgetrennt wird, wovon die schwere flüssige Phase zurück in den Absorber gefahren wird und/oder der Rücklauf im Absorber über eine Ablauftasse aus dem Absorber entnommen, in einem statischen Mischer mit dem flüssigen Lösemittel vermischt und dann mit dem flüssigen Lösemittel zu dem Flüssigkeitsverteiler oberhalb des ersten Füllkörperbettes zugeführt wird.

Ein Vorteil des vorliegenden Verfahrens ist der relativ einfache apparative Aufbau, durch den das Auftreten von einer zweiten flüssigen Phase und/oder das Risiko einer Schaumbildung verhindert werden kann. Dadurch wird die Trenneffizienz des Verfahrens verbessert und das Verfahren energetisch optimiert. Das erfindungsgemäße Verfahren ist außerdem durch eine effiziente Energierückgewinnung durch Wärmeintegration mit dem heißen Lösemittel gekennzeichnet.

Durch die Wärmeintegration wird dem Lösemittel Wärme entzogen. Der Grund dafür ist nicht nur, dass damit andere Ströme oder Kolonnen geheizt werden sollen, sondern in erster Linie die Abkühlung des Lösemittels für die Absorption. Die Absorption der Butene (hier: Schritt a) findet meistens bei einer geringeren Temperatur statt als die Desorption (hier: Schritt b). Wird dem Lösemittel bei der Wärmeintegration ausreichend Wärme entzogen, weist also eine geeignete Temperatur auf, kann das Lösemittel direkt in den Absorber gefahren werden. Es ist jedoch auch denkbar, dass das Lösemittel trotz der vorliegenden Wärmeintegration noch nicht die richtige Temperatur aufweist. In so einem Fall kann das Lösemittel nach der Wärmeintegration und vor dem Eintritt in den Absorber durch einen Restkühler geleitet werden, um auf eine geeignete Temperatur gekühlt zu werden.

Das vorliegende Verfahren betrifft die Abtrennung von Butenen aus butenhaltigen C4-Kohlenwasserstoffströmen. Diese Ströme enthalten üblicherweise neben den Butenen auch Alkane (n-Butan, Isobutan). Der Begriff Butane wird im Rahmen der vorliegenden Erfindung dabei so verstanden, dass er - sofern nichts anderes bezeichnet ist - sowohl n-Butan als auch Isobutan meint. Im erfindungsgemäßen Verfahren können daher alle C4-Kohlenwasserstoffströme eingesetzt werden, die zumindest Butene und Butane enthalten, sofern die Mengen, in denen die Butene und/oder Butane vorhanden sind, eine wirtschaftliche Durchführung des Verfahrens zulassen. In einer bevorzugten Ausführungsform der vorliegenden Erfindung besteht der eingesetzte C4-Kohlenwasserstoffstrom im Wesentlichen, d.h. zu mehr als 98 Gew.-%, vorzugsweise zu mehr als 99 Gew.-% aus Butanen und Butenen. Die entsprechenden Ströme können auch Verunreinigungen oder andere Kohlenwasserstoffe, wie 1 ,3-Butadien oder C5-Kohlenwasserstoffe, in geringen Mengen enthalten.

In dem erfindungsgemäßen Extraktionsverfahren wird ein flüssiges Lösemittel eingesetzt, in dem sich vorrangig die Butene des eingesetzten, gasförmigen C4-Kohlenwasserstoffstroms lösen. Geeignete Lösemittel sind aprotische Lösemittel, beispielsweise N-Methyl-2-pyrrolidon (NMP). Das erfindungsgemäße Verfahren wird vorzugsweise mit NMP als Lösemittel durchgeführt. In einer weiterhin bevorzugten Ausführungsform der vorliegenden Erfindung enthält das Lösemittel Wasser, insbesondere im Bereich von 1 bis 10 Gew.-%, vorzugsweise von 4 bis 9 Gew.-%, jeweils bezogen auf die Gesamtmenge an Lösemittel.

Als Absorber können insbesondere Füllkörperkolonnen dienen, die mindestens zwei Füllkörperbetten aufweisen. Solche Kolonnen sind dem Fachmann grundsätzlich bekannt. Oberhalb des ersten Füllkörperbettes befindet sich vorzugsweise eine Rückwaschzone mit mehreren theoretischen Böden, um das in die Gasphase mitgerissene Lösemittel zurückzuhalten. Oberhalb der Rückwaschzone ist der Kopf des Absorbers, wo ein im Vergleich zum eingesetzten C4-Kohlenwasserstoffstrom an Butanen angereicherter Strom anfällt. Über jedem der vorhandenen Füllkörperbetten befindet sich ein Flüssigkeitsverteiler, durch den die Flüssigkeit verteilt wird. Unter dem letzten Füllkörperbett würde der erfindungsgemäße Flüssigkeitssammler angeordnet sein, worunter sich der Sumpf des Absorbers befindet. Der exakte Aufbau des Absorbers hängt von verschiedenen Parametern ab und ist in gewisser Hinsicht variabel.

Das flüssige Lösemittel wird räumlich betrachtet oberhalb des Einlasses für den C4-Kohlenwasserstoffstroms zu einem Flüssigkeitsverteiler in den Absorber gegeben. In einer bevorzugten Ausführungsform wird das Lösemittel zu einem Flüssigkeitsverteiler oberhalb des ersten oder zweiten Füllkörperbettes und der C4-Kohlenwasserstoffstrom in ein oder mehrere Füllkörperbetten unterhalb des ersten oder zweiten Füllkörperbettes zum Absorber gegeben. Das flüssige Lösemittel wird im Absorber nach unten rieseln und mit dem (aufsteigenden) dampfförmigen C4-Kohlenwasserstoffstrom in Kontakt gebracht, wodurch ein Teil des C4-Kohlenwasserstoffstroms, der überwiegend Butene enthält, in das Lösemittel übergeht. In einer weiterhin bevorzugten Ausführungsform der vorliegenden Erfindung umfasst der Teil des C4-Kohlenwasserstoffstroms, der in das Lösemittel übergeht, mindestens 70 Gew.-%, besonders bevorzugt mindestens 80 Gew.-% Butene, bezogen auf die Zusammensetzung des in das Lösemittel übergegangenen Teils des C4-Kohlenwasserstoffstroms. Daraus ergibt sich insbesondere, dass mindestens 80%, besonders bevorzugt mindestens 90% der im eingesetzten C4-Kohlenwasserstoffstrom enthaltenen Butene in das Lösemittel übergehen.

Der C4-Kohlenwasserstoffstrom und das Lösemittel werden in Schritt a insbesondere im Gegenstrom miteinander in Kontakt gebracht. Die Temperatur im Sumpf des Absorbers beträgt dabei vorzugsweise zwischen 40 und 70 °C, besonders bevorzugt zwischen 45 und 65 °C. Der Kopfdruck im Absorber kann zwischen 3 und 7 bar absolut, vorzugsweise zwischen 4 und 6,5 bar absolut betragen.

Das beladene Lösemittel läuft im Absorber nach unten und wird in einem geeigneten Flüssigkeitssammler, insbesondere einem Kaminboden, gesammelt. Die Temperatur des im Flüssigkeitssammler anfallenden beladenen Lösemittels beträgt vorzugsweise zwischen 40 und 90°C, besonders bevorzugt zwischen 45 und 65 °C. Vom Flüssigkeitssammler wird das beladene Lösemittel abgenommen, durch einen Absorberverdampfer gefahren und dann unterhalb des Flüssigkeitssammlers in den Sumpf des Absorbers geleitet, wodurch überwiegend Butane aus dem beladenen Lösemittel ausgegast werden. Der Absorberverdampfer ist vorzugsweise ein Once-Through-Verdampfer, bei dem das beladene Lösemittel nur einmal durch den Verdampfer geleitet wird. Dadurch lassen sich möglichst niedrige Temperaturen realisieren, wodurch Fouling verhindert werden kann. Zudem wird die treibende Temperaturdifferenz vergrößert, was eine noch effizientere Energieausnutzung des NMP-Stroms ermöglicht. Der Absorberverdampfer kann auch mehrstufig ausgestaltet sein, d. h. es können mehrere Wärmetauscher bzw. mehrere Verdampfer vorhanden sein, die zum Absorberverdampfer gehören.

Im Sumpf verbleibt dann das vorwiegend mit Butenen beladene Lösemittel, welches von dort abgenommen und als Sumpfstrom zum Desorber geführt wird. Die Temperatur im Sumpfstrom des Absorbers, der zum Desorber geführt wird, beträgt dabei vorzugsweise zwischen 70 und 130 °C, besonders bevorzugt zwischen 85 und 120 °C.

Am Kopf des Absorbers fällt dann insbesondere ein im Vergleich zum eingesetzten C4-Kohlenwasserstoffstrom an Butanen angereicherter Strom an. Dieser Strom wird zumindest teilweise, vorzugsweise vollständig, in einer geeigneten Vorrichtung, beispielsweise einem Kondensator, kondensiert, wodurch ein Kondensat anfällt. Zumindest ein Teil des Kondensats wird dann als Rücklauf zum Absorber geführt. Der Rücklauf wird vorzugsweise oberhalb der Rückwaschzone des Absorbers zugeführt, von wo der Rücklauf in den Flüssigkeitsverteiler oberhalb des ersten Füllkörperbettes (unterhalb der Rückwaschzone) gelangt. Zum Flüssigkeitsverteiler oberhalb des ersten Füllkörperbettes kann kontinuierlich das flüssige Lösemittel zugeführt werden. An dieser Stelle oder auch in den darunter liegenden Füllkörperbetten kann es aufgrund der Butananteile im Rücklauf zum Auftreten einer zweiten flüssigen Phase kommen, die dann zur unerwünschten Schaumbildung führt.

An dieser Stelle setzt die vorliegende Erfindung an. Die flüssige Phase, die das flüssige Lösemittel und den Rücklauf zum Absorber bzw. die aus der Rückwaschzone zurücklaufende Flüssigkeit umfasst, kann am Flüssigkeitsverteiler oberhalb des ersten Füllkörperbettes abgenommen und zu einem Dekanter geleitet werden, wo eine Trennung in eine schwere flüssige Phase, die hauptsächlich NMP umfasst, und eine leichte flüssige Phase, die hauptsächlich Butane aus dem Rücklauf umfasst, stattfindet. Die schwere flüssige Phase wird dann zurück zum Absorber, insbesondere dem Flüssigkeitsverteiler im Absorber, gefahren. Die leichte flüssige Phase kann aus dem Verfahren herausgeführt, zum Desorber geführt oder einer weiteren Trennung unterworfen werden. Diese optionale ein- oder mehrstufige Trennung findet vor dem Eintritt in den Desorber statt. Eine Möglichkeit ist, dass die leichte flüssige Phase aufgeheizt und zu einem Flashbehälter geführt wird, wo eine flüssige Phase, die das flüssige Lösemittel umfasst, und eine gasförmige Phase, die Butane umfasst, anfallen. Nur die flüssige Phase wird dann zum Desorber geführt. Die gasförmige Phase wird aus dem Flashbehälter entnommen, kondensiert und kann dann aus dem Verfahren abgeführt werden, beispielsweise auch zusammen mit dem butanhaltigen Produktstrom.

Eine andere Möglichkeit ist, dass die die flüssige Phase am Flüssigkeitsverteiler oberhalb des zweiten Füllkörperbettes abgenommen und zu einem Dekanter geleitet wird. Das erste Füllkörperbett wird dabei "geopfert", d. h. es wir zugelassen, dass im ersten oder oberhalb des ersten Füllkörperbettes zum Auftreten einer dritten Phase kommt. Erst danach wird es durch die entsprechende Maßnahme, also das Durchfahren des Dekanters verhindert. Die oberhalb des zweiten Füllkörperbettes abgenommene flüssige Phase umfasst dabei das aus dem ersten Füllkörperbett stammende Gemisch.

Die Ausbildung der zweiten flüssigen Phase und die damit zusammenhängende Schaumbildung kann erfindungsgemäß auch auf eine andere Weise verringert oder verhindert werden. Erfindungsgemäß kann der Rücklauf zum Absorber, vorzugsweise vollständig, über eine Ablauftasse aus dem Absorber entnommen werden. Der Rücklauf kommt dabei nicht im Flüssigkeitsverteiler mit dem flüssigen Lösemittel in Kontakt. Stattdessen wird der Rücklauf in einem statischen Mischer mit dem flüssigen Lösemittel vermischt und dann mit dem flüssigen Lösemittel zum Absorber, insbesondere zum Flüssigkeitsverteiler oberhalb des ersten Füllkörperbettes des Absorbers, geführt. Die Verwendung eines solchen statischen Mischers führt unter anderem aufgrund der geringeren Konzentrationsunterschiede zwischen Rücklauf und NMP im Absorber zu einer geringeren Schaumbildungsneigung. Ein weiterer Vorteil ist dass die Schaumbildung dann wenn überhaupt außerhalb des Absorbers auftritt und dort besser damit umgegangen werden kann. Der statische Mischer kann sowohl vor als auch nach dem Restkühler für das zurückgeführte NMP angeordnet werden.

Die beiden hier vorgestellten Methoden, also die vorherige Mischung mit dem Lösemittel und das Durchfahren eines Dekanters, können auch miteinander kombiniert werden.

Der an Butanen angereicherte Strom kann zusätzlich Wasser enthalten, welches aus dem Lösemittel stammt. Dieses Wasser kann in einem nachfolgenden Schritt abgetrennt werden. Dabei wird der an Butanen angereicherte Strom am Kopf des Absorbers entnommen und einer ein- oder mehrstufigen Kondensation unterworfen, wobei ein wasserhaltiger Strom, der auch Butane enthält, und ein butanhaltiger Produktstrom auskondensiert werden. Diese beiden Ströme können in einer geeigneten Vorrichtung, beispielsweise einem Euter, voneinander getrennt werden. Der vom butanhaltigen Produktstrom abgetrennte wasserhaltige Strom kann je nach seiner Zusammensetzung zum Absorber oder zum Desorber geführt und/oder teilweise aus dem Verfahren ausgeschleust werden. Der butanhaltige Strom wird teilweise aus dem Verfahren ausgeschleust und teilweise als Rücklauf zum Absorber gefahren. Der Rücklauf zum Absorber meint im Rahmen dieser Erfindung also einen Teil des butanhaltigen Produktstroms.

Der so aus der Kondensation erhaltene butanhaltige Produktstrom kann noch geringe Mengen an Wasser enthalten, insbesondere in einer Menge von bis zu 1500 Gew.-ppm, bezogen auf die Gesamtzusammensetzung des butanhaltigen Produktstroms. Außerdem kann der aus der Kondensation erhaltene butanhaltige Produktstrom noch Restbutene enthalten, wobei die Ströme üblicherweise weniger als 20 Gew.-%, vorzugsweise weniger als 15 Gew.-%, besonders bevorzugt weniger als 5 Gew.-% an Butenen, bezogen auf die Gesamtzusammensetzung des butanhaltigen Produktstroms, enthalten.

Je nach den Anforderungen an den erhaltenen butanhaltigen Produktstrom kann es erforderlich sein, dass der butanhaltige Produktstrom nach der Kondensation einer Trocknung, vorzugsweise in einer Trocknungskolonne, unterworfen wird, um das noch enthaltene Wasser abzutrennen. Vorzugsweise weist der butanhaltige Produktstrom nach der Trocknung eine maximale Menge an Wasser von 50 Gew.-ppm, vorzugsweise von 25 Gew.-ppm auf. Das bei der Trocknung anfallende Wasser kann zur Kondensation am Absorber zurückgeführt werden.

Das im Sumpf des Absorbers abgenommene und vorwiegend mit Butenen beladenen Lösemittel wird dem Desorber zugeführt. Das beladene Lösemittel kann dazu beispielsweise mittels einer Pumpe zum Desorber geführt werden. Im Sumpf des Desorbers liegt im Vergleich zum Sumpf des Absorbers eine erhöhte Temperatur und bevorzugt ein geringerer Druck vor. Die Temperatur im Sumpf des Desorbers beträgt vorzugsweise zwischen 120 und 200 °C, weiterhin bevorzugt zwischen 130 und 195 °C. Der Kopfdruck im Desorber kann zwischen 1 und 6 bar absolut, vorzugsweise zwischen 2 und 5 bar absolut betragen. Durch die gegenüber dem Absorber erhöhte Temperatur und den vorzugsweise geringeren Druck werden die Butene und optional noch enthaltenen Butane zumindest teilweise aus dem Lösemittel entfernt. In einer bevorzugten Ausführungsform enthält das zumindest teilweise von Butenen befreite Lösemittel bis zu 5000 Gew.-ppm an Butenen, besonders bevorzugt 100 bis 900 Gew.-ppm an Butenen. Das zumindest teilweise von Butenen befreite Lösemittel läuft im Desorber nach unten und wird in einem Flüssigkeitssammler des Desorbers gesammelt. Von dort wird das zumindest teilweise von Butenen befreite Lösemittel durch einen Desorberverdampfer gefahren und dann unterhalb des Flüssigkeitssammlers, insbesondere einem Kaminboden, in den Sumpf des Desorbers geleitet, wodurch noch im Lösemittel vorhandene Butene ausgegast werden. Der Desorberverdampfer ist vorzugsweise ein Once-Through-Verdampfer, bei dem das zumindest teilweise von Butenen befreite Lösemittel nur einmal durch den Verdampfer geleitet wird. Dadurch lassen sich möglichst niedrige Temperaturen realisieren, wodurch Fouling verhindert werden kann. Der Desorberverdampfer kann auch mehrstufig ausgestaltet sein, d. h. es können mehrere Wärmetauscher vorhanden sein, die zum Desorberverdampfer gehören. Im Sumpf verbleibt dann das von Butenen befreite Lösemittel, welches von dort abgenommen, als Sumpfstrom zum Absorber geführt und dort wieder als Lösemittel für die Absorption von Butenen eingesetzt wird.

Das von Butenen befreite Lösemittel kann bevor es zum Absorber geführt wird teilweise oder vollständig einer Regeneration unterworfen werden, wodurch Verunreinigungen, beispielsweise die vorgenannt im eingesetzten C4-Kohlenwasserstoffstrom vorhandenen und/oder bei den Temperaturen im Desorber aus den Butenen gebildeten Nebenprodukte wie oligomere oder polymere Verbindungen, aus dem Lösemittel, vorzugsweise dem NMP, entfernt werden. Die Regeneration wird vorzugsweise so durchgeführt, dass das von Butenen befreite Lösemittel in einen Behälter gefahren und bei einem Druck von weniger als 500 mbar absolut, weiterhin bevorzugt von weniger als 200 mbar absolut und einer Temperatur zwischen 100 und 150 °C verdampft wird. An den Behälter kann eine Kolonne angeschlossen sein. Durch die Regeneration werden insbesondere Schwersieder abgetrennt. Wird nur ein Teil des von Butenen befreiten Lösemittels einer Regeneration unterworfen, wird der regenerierte Teil des Lösemittels anschließend mit dem nicht regenerierten Lösemittel vereint und zum Absorber zurückgeführt.

Am Kopf des Desorbers fällt dann insbesondere ein im Vergleich zu dem eingesetzten C4-Kohlenwasserstoffstrom an Butenen angereicherte Strom an. Dieser an Butenen angereicherte Strom kann zusätzlich Wasser enthalten, welches aus dem Lösemittel stammt. Dieses Wasser kann in einem nachfolgenden Schritt abgetrennt werden. Dabei wird der an Butenen angereichte Strom am Kopf des Desorbers entnommen und einer ein- oder mehrstufigen Kondensation unterworfen, wobei ein wasserhaltiger Strom, der neben Wasser auch noch Reste an Organik enthalten kann, und ein butenhaltiger Produktstrom auskondensiert werden. Diese beiden Ströme können in einer geeigneten Vorrichtung, beispielsweise einem Euter, voneinander getrennt werden. Das vom butenhaltigen Produktstrom abgetrennte wasserhaltige Strom kann anschließend zurück zum Desorber geführt werden. Möglich ist auch eine Ausschleusung des ganzen oder von Teilen des wasserhaltigen Stroms, um die Organik zu entfernen.

In einer bevorzugten Ausführungsform der vorliegenden Erfindung ist die Kondensation des am Kopf des Desorbers abgenommenen an Butenen angereicherten Stroms zweistufig ausgestaltet, wobei in einer ersten Stufe Wasser auskondensiert wird, das dann zum Desorber zurückgeführt wird, und in der zweiten Stufe der butenhaltige Produktstrom auskondensiert wird. Es kann jedoch auch sein, dass in der zweiten Stufe auch noch vorhandenes Wasser auskondensiert. Dieses restliche Wasser kann über eine geeignete Vorrichtung, beispielsweise einen Euter, vom butenhaltigen Produktstrom abgetrennt werden.

Der aus der Kondensation erhaltene butenhaltige Produktstrom enthält vorzugsweise weniger als 20 Gew.-%, weiterhin bevorzugt weniger als 16 Gew.-% an Butanen bezogen auf die Gesamtzusammensetzung des butenhaltigen Produktstroms. Der aus der Kondensation erhaltene butenhaltige Produktstrom weist stattdessen vorzugsweise einen Butengehalt von mindestens 70 Gew.-%, weiterhin bevorzugt von mindestens 75 Gew.-%, besonders bevorzugt von mindestens 86 Gew.-% bezogen auf die Gesamtzusammensetzung des butenhaltigen Produktstroms auf.

Ein kennzeichnendes Merkmal der vorliegenden Erfindung ist die Wärmeintegration unter Verwendung der Wärme des Lösemittels auf dem Weg vom Desorber zum Absorber und des Heißkondensats, welches im Desorberverdampfer anfällt. Die Wärme des als Sumpfstrom des Desorbers abgenommen Lösemittels, vorzugsweise des NMPs, wird erfindungsgemäß zur Wärmeintegration verwendet, in dem die Wärme des Lösemittels in jeweils mindestens einem Wärmetauscher für die Vorwärmung des zum Desorber geführten beladenen Lösemittels zur Verdampfung im Absorberverdampfer und zur Verdampfung des flüssigen C4-Kohlenwasserstoffstroms eingesetzt wird.

In einer bevorzugten Ausgestaltung der vorliegenden Erfindung wird die Vorwärmung des zum Desorber geführten beladenen Lösemittels zweistufig durchgeführt, wobei eine erste eine Wärmeübertragung auf das zum Desorber geführten beladenen Lösemittel in einem Wärmetauscher, beispielsweise einen Rohrbündelwärmetauscher, und eine zweite Wärmeübertragung auf das zum Desorber geführten beladenen Lösemittel in einem Kettleverdampfer erfolgt. Eine solche Ausgestaltung hat den Vorteil, dass bei der vorgenannten bevorzugten Ausgestaltung die Wärme, die in beiden Stufen, d. h. im Wärmetauscher und im Kettleverdampfer auf das beladene Lösemittel übertragen wird, aus dem als Sumpfstrom des Desorbers abgenommen Lösemittel als Wärmeträgermedium stammt. Die Verwendung eines Kettleverdampfers hätte auch den Vorteil, dass in der Leitung zum Desorber ein geringerer Vordruck herrschen könnte. Normalerweise ist ein hoher Vordruck notwendig, um das Verdampfen in der Rohrleitung zu verhindern, was zu Problemen bis zum Bersten der Rohrleitung führen könnte. Ein weiterer Vorteil ist, dass die Temperaturbelastung begrenzt und dadurch sichergestellt wird, dass die Temperaturdifferenz zur Wärmeübertragung ausreichend groß ist bzw. bleibt. Erfindungsgemäß wird das zumindest teilweise von Butenen befreite Lösemittel, vorzugsweise NMP, in einem Flüssigkeitssammler des Desorbers gesammelt und durch einen Desorberverdampfer gefahren, wodurch noch im Lösemittel vorhandene Butene ausgegast werden können. Die Wärme zur Verdampfung im Desorberverdampfer kann dabei in einem Wärmetauscher durch Wärmeübertragung von einem geeigneten Wärmeträgermedium eingetragen werden. Das Wärmeträgermedium kann insbesondere Heizdampf sein, der als Mittel- oder Hochdruckdampf eingesetzt wird. Als Heizdampf ist ein Mitteldruckdampf bevorzugt, welcher eine Temperatur von 150 bis 270 °C, vorzugsweise von 160 bis 250 °C aufweist. Der Mitteldruckdampf weist vorzugsweise einen Druck von 15 bis 30 bar absolut, besonders bevorzugt von 17 bis 25 bar absolut auf. Als Heizdampf kann auch ein Dampf mit einem Druck von > 30 bar absolut eingesetzt werden. Ein solcher Heizdampf kann auch als Hochdruckdampf bezeichnet werden.

Der zur Verdampfung eingesetzte Heizdampf kann im Wärmetauscher zumindest teilweise kondensieren, wodurch ein Heißkondensat bei einem Druck von 10 bis 20 bar absolut, vorzugsweise 12 bis 17 bar absolut und einer Temperatur von 150 bis 200 °C, vorzugsweise 160 bis 200 °C anfällt. Nach dem Wärmetauscher ist vorzugsweise ein Kondensatbehälter angeordnet, in dem das Heißkondensat vom Dampf getrennt werden kann. In dem Kondensatbehälter liegt vorzugsweise ein geringerer Druck vor als im Wärmetauscher. Aufgrund des geringeren Drucks kann ein Teil des Heißkondensats verdampfen, wodurch der gesamte Dampf, d. h. der nicht kondensierte Anteil des Heizdampfes und das im Kondensatbehälter durch Druckentspannung verdampfte Heißkondensat, im Kondensatbehälter als Niederdruckdampf anfällt. Niederdruckdampf weist vorliegend vorzugsweise einen Druck von mehr als 0 bar und weniger als 10 bar absolut auf. Die Temperatur des Niederdruckdampfs beträgt vorzugsweise 100 bis 180 °C.

Der dort anfallende Niederdruckdampf enthält noch Energie, die in keinem bekannten Verfahren ausgenutzt wird. Aus energetischer und wirtschaftlicher Sicht ist das jedoch nicht sinnvoll. Diese Energie kann jedoch in einer bevorzugten Ausgestaltung der vorliegenden Erfindung genutzt werden. Dazu kann der zur Verdampfung im Desorberverdampfer eingesetzte Heizdampf mittels eines, vorzugsweise regelbaren Dampfstrahlers (Thermokompressors) zur Verfügung gestellt werden. Der Thermokompressor wird dann sowohl mit dem eingesetzten Heizdampf, der beispielsweise aus einem entsprechenden Dampfnetz stammt, hier insbesondere der bevorzugt eingesetzte Mitteldruckdampf, als auch mit dem Niederdruckdampf aus dem Kondensatbehälter gespeist, wodurch ein Mischdampf entsteht, der dementsprechend das Wärmeträgermedium für den Desorberverdampfer ist. Der Mischdampf ist in dieser Ausgestaltung demnach der Heizdampf. Ein solcher Dampfstrahler ist so ausgestaltet, dass er mit einem Treibdampf betrieben wird und durch einen Unterdruck (Staudruck im Dampfstrahler) Saugdampf aus einem Behälter ansaugen kann, wodurch dann der Mischdampf entsteht, der als Wärmeträgermedium eingesetzt wird. Der Treibdampf ist im vorliegenden Fall der Heizdampf bzw. der Mitteldruckdampf, mit dem der Niederdruckdampf als Saugdampf aus dem Kondensatbehälter angesaugt und mit dem Treibdampf vermischt wird.

Der Vorteil einer solchen Ausgestaltung ist offensichtlich. Die Energie des im Kondensatbehälter anfallenden Niederdruckdampfes kann genutzt und damit Energie und Kosten gespart werden. Eine solche Verfahrensweise kann auch aus einem anderen Grund vorteilhaft sein. Der eingesetzte Dampfstrahler kann dahingehend regelbar sein, dass die Mengen von Mitteldruck- und Niederdruckdampf, beispielsweise in Abhängigkeit von bestimmten Prozessparametern, eingestellt werden können. Die Saugdampfmenge stellt sich dabei über die Treibdampfmenge ein. Die Mengen von Mitteldruck- und Niederdruckdampf können beispielsweise in Abhängigkeit von der Temperatur im Desorber eingestellt werden.

Eine weiterhin bevorzugte Ausgestaltung kann auch dann vorliegen, wenn der Desorber einen Seitenverdampfer aufweist. In einem solchen Fall kann das Wärmeträgermedium, welches für den Seitenverdampfer eingesetzt wird, der Mischdampf aus dem Dampfstrahler sein, während im Desorberverdampfer Mitteldruckdampf als Heizdampf eingesetzt wird. Das Heißkondensat aus dem Desorberverdampfer und dem Seitenverdampfer werden dann entsprechend den obigen Ausführungen zu einem Kondensatbehälter geleitet. Der dort anfallende Niederdruckdampf wird dann im Dampfstrahler verwendet, dessen Mischdampf im Seitenverdampfer eingesetzt wird. Der Vorteil dieser Variante ist, dass das anfallende Heißkondensat weiter entspannt werden könnte, um eine größere Niederdruckdampfmenge bereitstellen zu können.

Das vorliegend beschriebene Verfahren kann in chemischen Verbünden, die insbesondere eine Oligomerisierung und optional eine Hydroformylierung umfassen, eingesetzt werden. Dabei ist es möglich, dass die Abtrennung von Butenen nach dem erfindungsgemäßen Verfahren an verschiedenen Stellen im Verbund eingesetzt wird. Es ist auch möglich, wenn die erfindungsgemäße Abtrennung von Butenen an mehreren Stellen innerhalb eines chemischen Verbundes vorhanden ist. Möglich ist beispielsweise, dass das hier beschriebene Verfahren zu Beginn eines solchen Verbunds eingesetzt wird. Der eingesetzte C4-Kohlenwasserstoffstrom kann dann insbesondere ein Crack-C4, ein Raffinat 1, ein Raffinat 2 oder eine Mischung daraus sein. Sofern Crack-C4 und/oder das Raffinat 2 eingesetzt werden, kann vor dem erfindungsgemäßen Abtrennverfahren eine Crack-C4-Hydrierung, in der Butadien selektiv hydriert wird, oder eine Butadienabtrennung, bei der Butadien extraktiv mit einem Lösemittel wie NMP oder Nitrilen entfernt wird, erfolgen, um den Gehalt an Butadien zu reduzieren. Nach einer extraktiven Butadienabtrennung und vor der erfindungsgemäßen Abtrennung kann eine Hydroisomerisierung angeordnet sein, um die Trennaufgabe beim erfindungsgemäßen Verfahren zu erleichtern, da 1-Buten in 2-Buten umgewandelt wird, was vom Lösemittel in der Regel besser aufgenommen wird. Wird das Abtrennverfahren zu Beginn des Verbunds eingesetzt, kann der erhaltene Produktstrom einer MTBE-Synthese zugeführt werden und vorzugsweise anschließend sukzessive eine 1-Butenabtrennung, eine Oligomerisierung und eine oder mehrere Hydroformylierung(en) auf die aufgereinigten Oligomere erfolgen. Eine Hydroformylierung kann sowohl mit dem Produktstrom aus der Oligomerisierung erfolgen, wodurch zum Beispiel aus Di-n-butenen nach anschließender Hydrierung INA (Isononanol) oder aus Tributenen ITDA (Isotridecanal) hergestellt werden kann, als auch mit den nicht umgesetzten Butenen der Oligomerisierung, wodurch nach anschließender Aldolkondensation und nachfolgender Hydrierung 2-PH (2-Propylheptanol) hergestellt werden kann. Mit den nicht umgesetzten Butenen aus der Oligomerisierung könnte ggf. auch anstatt einer Hydroformylierung eine weitere Oligomerisierung betrieben werden. Die Bedingungen der einzelnen Verfahrensschritte sind dem Fachmann geläufig. Die einzelnen Verfahrensschritte können weitere Schritte wie beispielsweise die Abtrennung der Produkte oder die Aufarbeitung der resultierenden Ströme enthalten, sind hier jedoch nicht explizit genannt. Das erfindungsgemäße Abtrennverfahren kann aber auch an jeder anderen Stelle eines solchen Verbunds eingefügt werden.

In einer Ausführungsform der vorliegenden Erfindung wird der beim erfindungsgemäßen Abtrennverfahren eingesetzte C4-Kohlenwasserstoffstrom einer MTBE-Synthese nach der Abtrennung von MTBE entnommen und der butenhaltige Produktstrom anschließend einer 1-Butenabtrennung zugeführt, wonach sukzessive eine Oligomerisierung und eine oder mehrere Hydroformylierung(en) zur anschließenden Herstellung von 2-PH und/oder INA erfolgen. Die einzelnen Verfahrensschritte können weitere Schritte wie beispielsweise die Abtrennung der Produkte oder die Aufarbeitung der resultierenden Ströme enthalten, sind hier jedoch nicht explizit genannt.

In einer Ausführungsform der vorliegenden Erfindung wird der beim erfindungsgemäßen Abtrennverfahren eingesetzte C4-Kohlenwasserstoffstrom einer MTBE-Synthese nach der Abtrennung von MTBE entnommen und der butenhaltige Produktstrom anschließend einer 1-Butenabtrennung zugeführt, wonach sukzessive eine Oligomerisierung und eine oder mehrere Hydroformylierung(en) zur anschließenden Herstellung von 2-PH, ITDA und/oder INA erfolgen. Die einzelnen Verfahrensschritte können weitere Schritte wie beispielsweise die Abtrennung der Produkte oder die Aufarbeitung der resultierenden Ströme enthalten, sind hier jedoch nicht explizit genannt.

In einer weiteren Ausführungsform der vorliegenden Erfindung wird der beim erfindungsgemäßen Abtrennverfahren eingesetzte C4-Kohlenwasserstoffstrom einer 1-Butenabtrennung entnommen und der butenhaltige Produktstrom anschließend einer Oligomerisierung zugeführt, wonach eine oder mehrere Hydroformylierung(en) zur anschließenden Herstellung von 2-PH, ITDA und/oder INA erfolgen. Die einzelnen Verfahrensschritte können weitere Schritte wie beispielsweise die Abtrennung der Produkte oder die Aufarbeitung der resultierenden Ströme enthalten, sind hier jedoch nicht explizit genannt.

In einer weiteren Ausführungsform der vorliegenden Erfindung wird das erfindungsgemäße Abtrennverfahren am Ende des Verbunds eingesetzt. In dem Fall wird der eingesetzte C4-Kohlenwasserstoffstrom einer der Hydroformylierung sich anschließenden 2-PH-Herstellung entnommen. Der aus dem erfindungsgemäßen Abtrennverfahren sodann erhaltene butenhaltige Produktstrom kann in diesem Fall zurückgeführt werden und an einer geeigneten Stelle im Verbund, beispielsweise zur 1-Butenabtrennung, zur Oligomerisierung oder einer oder mehrere Hydroformylierung(en) eingesetzt werden. Dadurch kann die Effizienz des gesamten Verbunds gesteigert werden, da selbst nach Durchlaufen des letzten Verfahrensschrittes im Verbund noch bis zu 20 Gew.-% Butene vorhanden sein können.

Der butanhaltige Produktstrom kann unabhängig von der Stelle im Verbund, in der das erfindungsgemäße Abtrennverfahren angeordnet ist, beispielsweise einer adiabaten Oligomerisierung, einer Hydrierung der noch vorhandenen Butene oder einer n/-iso-Splittung der Butane, bei der n-Butan und Isobutan voneinander getrennt werden, zugeführt werden. Die n/iso-Splittung kann auch nach einer adiabaten Oligomerisierung erfolgen. Möglich wäre auch eine Einbindung des butanhaltigen Produktstroms vor der Oligomerisierung in einem oben beschriebenen Verbund aus MTBE-Synthese, 1-Butenabtrennung, Oligomerisierung und einer Hydroformylierung.

In einer besonders bevorzugten Ausführungsform der vorliegenden Erfindung kann die zur n/iso-Splittung erforderliche Energie zumindest teilweise durch Wärmeintegration mit der ersten Stufe einer zweistufigen Kondensation am Kopf des Desorbers erfolgen. Das hat den Vorteil, dass die Energie, die in der Kondensation anfällt, genutzt wird und nicht wie im Stand der Technik einfach an die Umgebung abgegeben wird.

Die vorliegende Erfindung wird nachfolgend anhand von Abbildungen erläutert. Die Abbildungen dienen der Veranschaulichung, sind aber nicht einschränkend zu verstehen.

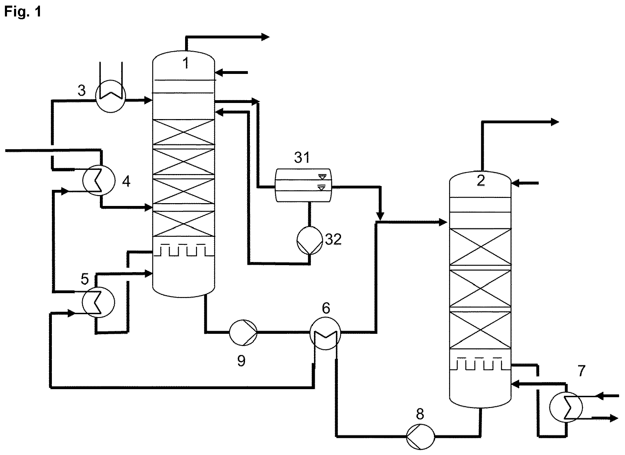

Fig. 1 zeigt die grundsätzliche Ausgestaltung der vorliegenden Erfindung. Der flüssige C4-Kohlenwasserstoffstrom wird über einen Wärmetauscher (4) verdampft und in den Absorber (1) geleitet. Das Lösemittel wird über einen Restkühler (3) - wenn notwendig - auf die gewünschte Temperatur gebracht und ebenfalls in den Absorber geleitet, wobei der Einlass räumlich gesehen oberhalb des Einlasses für den C4-Kohlenwasserstoffstrom ist, im vorliegenden Fall oberhalb des ersten Füllkörperbettes zu einem Flüssigkeitsverteiler (nicht eingezeichnet). Von dort wird die flüssige Phase entnommen und zum Dekanter (31) geführt. Die leichte flüssige Phase wird zum Desorber (hier zusammen mit dem beladenen Lösemittel) und die schwere flüssige Phase über eine Pumpe (32) zum Absorber (1) zurückgeleitet. Am Kopf des Absorbers (1) fällt der an Butanen angereicherte Strom an, der abgenommen wird. Eine mögliche Kondensation ist hier nicht gezeigt, lediglich der Rücklauf ist angedeutet. Im Sumpf des Absorbers (1) wird das beladene Lösemittel gesammelt, was in der Abbildung durch den Kaminboden angedeutet ist. Dort wird zumindest ein Teil des beladenen Lösemittels abgenommen und über einen Absorberverdampfer (5) zum Sumpf des Absorbers (1) geführt. Aus dem Sumpf des Absorbers (1) wird das beladene Lösemittel abgenommen und mittels einer Pumpe (9) über den Wärmetauscher (6) zur Vorwärmung des beladenen Lösemittels zum Desorber (2) geleitet, wo die im Lösemittel vorhandenen Butene vom Lösemittel getrennt werden. Am Kopf des Desorbers fällt der an Butenen angereicherte Strom an. Dieser Strom kann einer ein- oder mehrstufigen Kondensation unterworfen werden, was in der Abbildung nicht gezeigt ist. Lediglich ein möglicher Rückführstrom ist angedeutet. Im Sumpf des Desorbers (2) wird das zumindest teilweise von Butenen befreite Lösemittel gesammelt, was in der Abbildung durch den Kaminboden angedeutet ist. Dort wird zumindest ein Teil des beladenen Lösemittels abgenommen und über einen Desorberverdampfer (7) zum Sumpf des Desorbers (2) geführt. Aus dem Sumpf des Desorbers (2) wird dann das von Butenen befreite Lösemittel abgenommen und mittels einer Pumpe (8) über den Wärmetauscher (6) zur Vorwärmung des beladenen Lösemittels, den Absorberverdampfer (5), den Wärmetauscher (4) zur Verdampfung des C4-Kohlenwasserstoffstroms und den Restkühler (3) zum Absorber zurückgeführt.

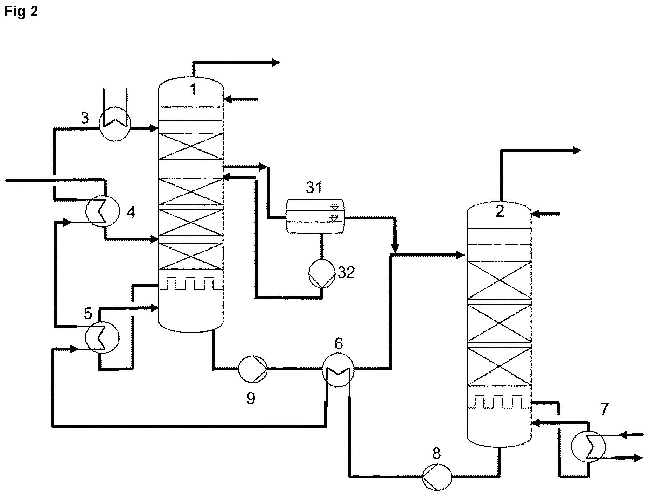

Fig. 2 zeigt eine Ausführungsform, die in weiten Teilen mit der in Fig. 1 dargestellten Ausführungsform identisch ist. Der Unterschied besteht darin, dass die flüssige Phase vom Flüssigkeitsverteiler (nicht eingezeichnet) oberhalb des zweiten Füllkörperbettes entnommen und zum Dekanter (31) geführt wird.

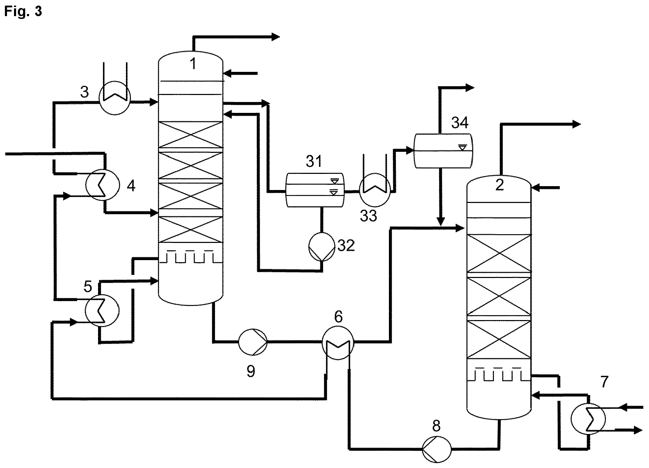

Fig. 3 zeigt eine bevorzugte Ausführungsform der vorliegenden Erfindung, bei der die leichte flüssige Phase zunächst in einer geeigneten Heizvorrichtung (33) erhitzt und anschließend einem Flashbehälter (34) zugeführt wird. Die gasförmige Phase wird abgeführt, wobei die unterschiedlichen Möglichkeiten nicht eingezeichnet sind. Die flüssige Phase aus dem Flashbehälter (34) wird zum Desorber (2) geführt, hier zusammen mit dem beladenen Lösemittel.

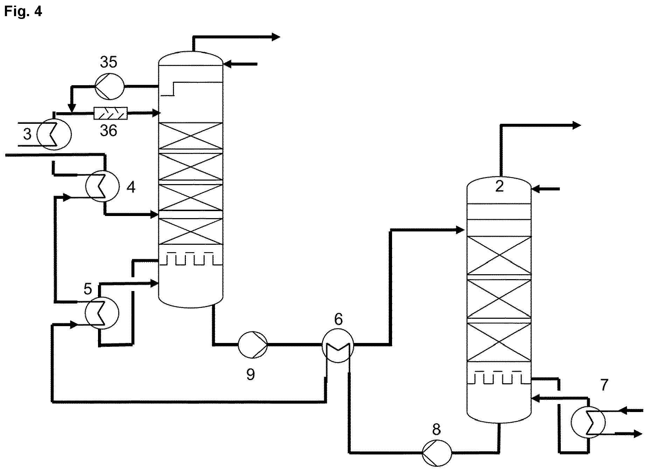

Fig. 4 zeigt die weitere erfindungsgemäße Ausführungsform. Der Rücklauf wird über eine Ablauftasse abgenommen und über eine Pumpe (35) zu einem statischen Mischer (36), der nach dem Restkühler (3) angeordnet ist, geführt, wo der Rücklauf mit dem flüssigen Lösemittel vermischt wird und dann gemeinsam mit dem flüssigen Lösemittel zum Absorber (1) geführt wird. Alternativ kann der statische Mischer (36) schon vor dem Restkühler (3), also direkt nach dem Wärmetauscher (4) angeordnet sein.

## Patentansprüche

1. Verfahren zur Abtrennung von Butenen aus einem C4-Kohlenwasserstoffstrom, der zumindest Butene und Butane enthält, durch Extraktivdestillation mit einem Lösemittel, wobei das Verfahren die folgenden Schritte umfasst:
a. Zumindest teilweises Verdampfen des flüssigen C4-Kohlenwasserstoffstroms in einem Feedverdampfer, Zuführen des gasförmigen C4-Kohlenwasserstoffstroms und Zuführen des flüssigen Lösemittels zu einem Flüssigkeitsverteiler oberhalb des C4-Kohlenwasserstoffstroms zu einem Absorber, der mehr als zwei Füllkörperbetten umfasst und in dem der C4-Kohlenwasserstoffstrom und das Lösemittel miteinander in Kontakt gebracht werden, wodurch überwiegend Butene aus dem C4-Kohlenwasserstoffstrom in das Lösemittel übergehen, wobei das so beladene Lösemittel in einem Flüssigkeitssammler des Absorbers gesammelt, durch einen Absorberverdampfer gefahren und dann unterhalb des Flüssigkeitssammlers in den Sumpf des Absorbers geleitet wird, wodurch überwiegend Butane aus dem beladenen Lösemittel ausgegast werden, wobei das beladene Lösemittel anschließend als Sumpfstrom zu einem Desorber geführt wird und wobei am Kopf des Absorbers ein im Vergleich zum eingesetzten C4-Kohlenwasserstoffstrom an Butanen angereicherter Strom anfällt, der zumindest teilwiese kondensiert und ein Teil des Kondensats als Rücklauf zum Kopf des Absorbers geleitet wird;
b. Zuführen des Zuführen des beladenen Lösemittels, vorzugsweise NMP, zu dem Desorber, in dem im Vergleich mit dem Absorber eine erhöhte Temperatur und bevorzugt ein geringerer Druck vorliegt und in dem die Butene vom Lösemittel, vorzugsweise NMP, getrennt werden, wodurch am Kopf des Desorbers ein an Butenen angereicherter Strom und im Sumpf des Desorbers das zumindest teilweise von Butenen befreite Lösemittel, vorzugsweise NMP, anfällt, wobei das zumindest teilweise von Butenen befreite Lösemittel, vorzugsweise NMP, in einem Flüssigkeitssammler des Desorbers gesammelt, durch einen Desorberverdampfer gefahren und dann unterhalb des Flüssigkeitssammlers in den Sumpf des Desorbers geleitet wird, wodurch noch im Lösemittel vorhandene Butene ausgegast werden, und wobei das Lösemittel, vorzugsweise NMP, anschließend als Sumpfstrom zum Absorber zurückgeführt wird;
**dadurch gekennzeichnet, dass** die Wärme des als Sumpfstrom des Desorbers abgenommen Lösemittels zur Wärmeintegration verwendet wird, in dem die Wärme des Lösemittels in jeweils mindestens einem Wärmetauscher für die Vorwärmung des zum Desorber geführten beladenen Lösemittels, zur Verdampfung im Absorberverdampfer und zur Verdampfung des flüssigen C4-Kohlenwasserstoffstroms eingesetzt wird, und dass
zumindest ein Teil einer flüssigen Phase am Flüssigkeitsverteiler oberhalb des ersten oder zweiten Füllkörperbettes abgenommen und in einem Dekanter in eine schwere flüssige Phase und eine leichte flüssige Phase aufgetrennt wird, wovon die schwere flüssige Phase zurück in den Absorber gefahren wird und/oder der Rücklauf im Absorber über eine Ablauftasse aus dem Absorber entnommen, in einem statischen Mischer mit dem flüssigen Lösemittel vermischt und dann mit dem flüssigen Lösemittel zu dem Flüssigkeitsverteiler oberhalb des ersten Füllkörperbettes zugeführt wird.

2. Verfahren nach Anspruch 1 oder 2, wobei das eingesetzte Lösemittel NMP ist.

3. Verfahren nach Anspruch 1 oder 2, wobei das Lösemittel bzw. das NMP Wasser enthält und der Wassergehalt zwischen 1 und 10 Gew.-%, vorzugsweise zwischen 4 und 9 Gew.-% liegt.

4. Verfahren nach einem der vorhergehenden Ansprüche, wobei der an Butenen angereicherte Strom, der am Kopf des Desorbers anfällt, zusätzlich Wasser enthält, welches aus dem Lösemittel stammt.

5. Verfahren nach Anspruch 5, wobei der an Butenen angereichter Strom am Kopf des Desorbers entnommen und einer Kondensation unterworfen wird, wobei Wasser und butenhaltiger Produktstrom auskondensiert und voneinander getrennt werden und das Wasser zurück zum Absorber geführt wird.

6. Verfahren nach Anspruch 6, wobei der aus der Kondensation erhaltene butenhaltige Produktstrom einen Butengehalt von mindestens 70 Gew.-%, vorzugsweise mindestens 75 Gew.-%, besonders bevorzugt mindestens 86 Gew.-% bezogen auf die Gesamtzusammensetzung des butenhaltigen Produktstroms, aufweist.

7. Verfahren nach einem der vorhergehenden Ansprüche, wobei die schwere flüssige Phase mittels einer Pumpe vom Dekanter zum Absorber geführt wird.

8. Verfahren nach einem der vorhergehenden Ansprüche, wobei die leichte flüssige Phase, die am Dekanter anfällt, zum Desorber geführt wird.

9. Verfahren nach Anspruch 8, wobei die leichte flüssige Phase vorher aufgeheizt und zu einem Flashbehälter geführt wird, wo eine flüssige Phase und eine gasförmige Phase anfallen, wovon die flüssige Phase zum Desorber geführt wird.

10. Verfahren nach Anspruch 9, wobei die gasförmige Phase aus dem Flashbehälter kondensiert und aus dem Verfahren abgeführt wird.

11. Verfahren nach einem der vorhergehenden Ansprüche, wobei die Vorwärmung des zum Desorber geführten beladenen Lösemittels zweistufig durchgeführt wird, wobei eine erste eine Wärmeübertragung auf das Lösemittel in einem Wärmetauscher und eine zweite Wärmeübertragung auf das Lösemittel in einem Kettleverdampfer erfolgt.

12. Verfahren nach einem der vorhergehenden Ansprüche, wobei die Wärme zur Verdampfung im Desorberverdampfer durch Wärmeaustausch in einem Wärmetauscher mit einem geeigneten Wärmeträgermedium, insbesondere Heizdampf, eingetragen wird.

13. Verfahren nach einem der vorhergehenden Ansprüche, wobei der eingesetzte Heizdampf im Wärmetauscher zumindest teilweise kondensiert und dadurch ein Heißkondensat bei einem Druck von 10 bis 20 bar absolut, vorzugsweise 12 bis 17 bar absolut und einer Temperatur von 150 bis 200 °C, vorzugsweise 160 bis 190 °C anfällt, das zu einem Kondensatbehälter geleitet wird.

14. Verfahren nach Anspruch 13, wobei im Kondensatbehälter ein geringerer Druck vorliegt als im Wärmetauscher und dadurch ein Teil des Heißkondensats wieder verdampft, wodurch der gesamte Dampf als Niederdruckdampf anfällt.

15. Verfahren nach einem der vorhergehenden Ansprüche, wobei der Heizdampf für den Desorberverdampfer mittels eines Dampfstrahlers, der mit Mitteldruckdampf und dem im Kondensatbehälter anfallenden Niederdruckdampf gespeist wird, zur Verfügung gestellt wird.

## Claims

1. Process for separating butenes from a C4-hydrocarbon stream which contains at least butenes and butanes by extractive distillation with a solvent, wherein the process comprises the steps of:
a. at least partially evaporating the liquid C4-hydrocarbon stream in a feed evaporator, supplying the gaseous C4-hydrocarbon stream and supplying the liquid solvent to a liquid distributor above the C4-hydrocarbon stream to an absorber which comprises more than two random-packing beds and in which the C4-hydrocarbon stream and the solvent are contacted with one another to transfer predominantly butenes from the C4-hydrocarbon stream to the solvent, wherein the thus-laden solvent is collected in a liquid collector of the absorber, passed through an absorber evaporator and then passed into the bottom of the absorber below the liquid collector to outgas predominantly butanes from the laden solvent, wherein the laden solvent is subsequently passed to a desorber as bottoms stream and wherein a stream enriched in butanes relative to the employed C4-hydrocarbon stream is obtained at the top of the absorber and at least partially condensed and a portion of the condensate is returned to the top of the absorber as reflux;
b. supplying the supplying the laden solvent, preferably NMP, to the desorber, which is at an elevated temperature and preferably a lower pressure relative to the absorber and in which the butenes are separated from the solvent, preferably NMP, to obtain at the top of the desorber a stream enriched in butenes and to obtain at the bottom of the desorber the solvent at least partially freed of butenes, preferably NMP, wherein the solvent at least partially freed of butenes, preferably NMP, is collected in a liquid collector of the desorber, passed through a desorber evaporator and then passed into the bottom of the desorber below the liquid collector to outgas any butenes remaining in the solvent and wherein the solvent, preferably NMP, is subsequently recycled to the absorber as bottoms stream;
**characterized in that** the heat of the solvent withdrawn as a bottoms stream of the desorber is used for heat integration by employing the heat of the solvent in at least one respective heat exchanger for preheating the laden solvent passed to the desorber, for evaporation in the absorber evaporator and for evaporation of the liquid C4-hydrocarbon stream and **in that**
at least a portion of a liquid phase is withdrawn at the liquid distributor above the first or second random-packing bed and separated into a heavy liquid phase and a light liquid phase in a decanter, of which the heavy liquid phase is returned to the absorber and/or the reflux in the absorber is withdrawn from the absorber via a runoff cup, mixed with the liquid solvent in a static mixer and then, with the liquid solvent, supplied to the liquid distributor above the first random-packing bed.

2. Process according to Claim 1 or 2, wherein the employed solvent is NMP.

3. Process according to Claim 1 or 2, wherein the solvent/the NMP contains water and the water content is between 1% and 10% by weight, preferably between 4% and 9% by weight.

4. Process according to any of the preceding claims, wherein the stream enriched in butenes obtained at the top of the desorber additionally contains water originating from the solvent.

5. Process according to Claim 5, wherein the stream enriched in butenes is withdrawn at the top of the desorber and subjected to a condensation, wherein water and butene-containing product stream are condensed out and separated from one another and the water is recycled to the absorber.

6. Process according to Claim 6, wherein the butene-containing product stream obtained from the condensation has a butene content of at least 70% by weight, preferably at least 75% by weight, particularly preferably at least 86% by weight, based on the total composition of the butene-containing product stream.

7. Process according to any of the preceding claims, wherein the heavy liquid phase is passed from the decanter to the absorber using a pump.

8. Process according to any of the preceding claims, wherein the light liquid phase obtained at the decanter is passed to the desorber.

9. Process according to Claim 8, wherein the light liquid phase is first heated and passed to a flash vessel where a liquid phase and a gaseous phase are obtained, of which the liquid phase is passed to the desorber.

10. Process according to Claim 9, wherein the gaseous phase from the flash vessel is condensed and discharged from the process.

11. Process according to any of the preceding claims, wherein the preheating of the laden solvent passed to the desorber is performed in two stages, wherein a first heat transfer to the solvent is effected in a heat exchanger and a second heat transfer to the solvent is effected in a kettle evaporator.

12. Process according to any of the preceding claims, wherein the heat for evaporation in the desorber evaporator is introduced in a heat exchanger by heat transfer with a suitable heat transfer medium, in particular heating steam.

13. Process according to any of the preceding claims, wherein the employed heating steam undergoes at least partial condensation in the heat exchanger, thus generating a hot condensate at a pressure of 10 to 20 bar absolute, preferably 12 to 17 bar absolute, and a temperature of 150°C to 200°C, preferably 160°C to 190°C, which is passed to a condensate container.

14. Process according to Claim 13, wherein the pressure in the condensate container is lower than in the heat exchanger, thus causing a portion of the hot condensate to be re-evaporated, as a result of which the combined steam is obtained as low pressure steam.

15. Process according to any of the preceding claims, wherein the heating steam for the desorber evaporator is provided using a steam ejector supplied with medium pressure steam and the low pressure steam obtained in the condensate container.

## Revendications

1. Procédé de séparation de butènes d'un courant d'hydrocarbures en C4 qui contient au moins des butènes et des butanes, par distillation extractive avec un solvant, le procédé comprenant les étapes suivantes :
a. évaporation au moins partielle du courant d'hydrocarbures liquides en C4 dans un évaporateur à injection, amenée du courant d'hydrocarbures gazeux en C4 et amenée du solvant liquide à un répartiteur de liquide au-dessus du courant d'hydrocarbures en C4 jusqu'à un absorbeur qui comprend plus de deux lits à garnissage, et dans lequel le courant d'hydrocarbures en C4 et le solvant sont mis en contact l'un avec l'autre, ce en conséquence de quoi il y a essentiellement transfert des butènes du courant d'hydrocarbures en C4 dans le solvant, le solvant ainsi chargé étant recueilli dans le réservoir de liquide de l'absorbeur, envoyé dans un évaporateur-absorbeur puis guidé en dessous du réservoir de liquide au fond de l'absorbeur, ce qui évacue essentiellement les butanes du solvant chargé, le solvant chargé étant ensuite envoyé sous forme d'un courant de fond à un désorbeur, et avec obtention, en tête de l'absorbeur, d'un courant enrichi en butanes par rapport au courant d'hydrocarbures en C4 utilisé, courant qui est au moins partiellement condensé, et une partie du condensat étant envoyée en reflux en tête de l'absorbeur ;
b. amenée du solvant chargé, de préférence du NMP, au désorbeur, dans lequel règnent, par rapport à l'absorbeur, une température plus élevée et une pression plus faible, et dans lequel les butènes sont séparés du solvant, de préférence le NMP, avec obtention en tête du désorbeur d'un courant enrichi en butènes et au fond du désorbeur, du solvant au moins partiellement débarrassé des butènes, de préférence le NMP, le solvant au moins partiellement débarrassé des butènes, de préférence le NMP, étant recueilli dans un réservoir de liquide du désorbeur, envoyé dans un évaporateur-désorbeur puis guidé en dessous du réservoir de liquide dans le réservoir du désorbeur, les butènes encore présents dans le solvant étant alors évacués sous forme gazeuse, et le solvant, de préférence le NMP, étant ensuite renvoyé en tant que courant de fond à l'absorbeur ;
**caractérisé en ce que** la chaleur du solvant soutiré en tant que courant de fond du désorbeur est utilisée pour intégration de la chaleur, au cours de laquelle la chaleur du solvant est, dans chaque cas dans au moins un échangeur de chaleur, utilisée pour le préchauffage du solvant chargé envoyé au désorbeur, pour l'évaporation dans l'évaporateur-absorbeur et pour l'évaporation du courant d'hydrocarbures liquides en C4, et **en ce que**
au moins une partie d'une phase liquide dans le répartiteur de liquide est soutirée au-dessus du premier ou du deuxième lit à garnissage et est séparée dans un décanteur en une phase liquide lourde et une phase liquide légère, la phase liquide étant renvoyée à l'absorbeur et/ou le reflux se trouvant dans l'absorbeur étant, par un bac de trop-plein, prélevé de l'absorbeur, mélangé dans un mélangeur statique au solvant liquide, puis, avec le solvant liquide, envoyé vers le répartiteur de liquide au-dessus du premier lit à garnissage.

2. Procédé selon la revendication 1 ou 2, dans lequel le solvant utilisé est le NMP.

3. Procédé selon la revendication 1 ou 2, dans lequel le solvant ou le NMP contient de l'eau, et la teneur en eau est comprise entre 1 et 10 % en poids, de préférence entre 4 et 9 % en poids.

4. Procédé selon l'une des revendications précédentes, dans lequel le courant enrichi en butènes obtenu en tête du désorbeur contient en outre de l'eau, qui provient du solvant.

5. Procédé selon la revendication 5, dans lequel le courant enrichi en butènes est soutiré en tête du désorbeur et soumis à une condensation, l'eau et le courant de produit contenant du butène étant isolés par condensation et séparés l'un de l'autre, et l'eau étant renvoyée à l'absorbeur.

6. Procédé selon la revendication 6, dans lequel le courant de produit contenant du butène, obtenu par la condensation, présente une teneur en butène d'au moins 70 % en poids, de préférence d'au moins 75 % en poids, d'une manière particulièrement préférée d'au moins 86 % en poids par rapport à la composition totale du courant de produit contenant du butène.

7. Procédé selon l'une des revendications précédentes, dans lequel la phase liquide lourde est envoyée du décanteur à l'absorbeur à l'aide d'une pompe.

8. Procédé selon l'une des revendications précédentes, dans lequel la phase liquide légère obtenue au décanteur est envoyée au désorbeur.

9. Procédé selon la revendication 8, dans lequel la phase liquide légère est d'abord chauffée et envoyée dans un évaporateur éclair, où se forment une phase liquide et une phase gazeuse, dont la phase liquide est envoyée au désorbeur.

10. Procédé selon la revendication 9, dans lequel la phase gazeuse est condensée à partir de l'évaporateur éclair et évacuée du procédé.

11. Procédé selon l'une des revendications précédentes, dans lequel le préchauffage du solvant chargé envoyé au désorbeur est réalisé en deux étapes, avec un premier transfert de chaleur au solvant dans un échangeur de chaleur et un deuxième transfert de chaleur au solvant dans un évaporateur tubulaire à chaudière.

12. Procédé selon l'une des revendications précédentes, dans lequel la chaleur destinée à l'évaporation dans l'évaporateur-désorbeur est injectée par échange de chaleur dans un échangeur de chaleur contenant un fluide caloporteur approprié, en particulier de la vapeur de chauffage.

13. Procédé selon l'une des revendications précédentes, dans lequel la vapeur de chauffage utilisée est au moins partiellement condensée dans l'échangeur de chaleur, avec obtention d'un condensat chaud sous une pression de 10 à 20 bar absolus, de préférence de 12 à 17 bar absolus et à une température de 150 à 200 °C, de préférence de 160 à 190 °C, qui est envoyé à un réservoir de condensats.

14. Procédé selon la revendication 13, dans lequel règne dans le réservoir de condensats une pression plus faible que dans l'échangeur de chaleur, ce qui provoque une nouvelle évaporation d'une partie du condensat chaud, la totalité de la vapeur étant obtenue sous forme d'une vapeur basse pression.

15. Procédé selon l'une des revendications précédentes, dans lequel la chaleur de chauffage dans l'évaporateur-désorbeur est mise à disposition à l'aide d'un jet de vapeur, qui est alimenté en vapeur moyenne pression et qui alimente la vapeur basse pression obtenue dans le réservoir de condensats.
